# EUROPEAN PATENT APPLICATION

(11) **EP 4 438 730 A1**
(43) Date of publication of application: **02.10.2024**
(21) Application number: 22895742.9
(22) Date of filing: 22.11.2022
(51) Int. Cl.: C12N 15/55, A23L 5/00, C12N 1/15, C12N 1/19, C12N 1/21, C12N 5/10, C12N 9/14, C12N 15/63

(54) **PROTEIN-DEAMIDATING ENZYME**

(30) Priority: 22.11.2021 JP 2021189716
(71) Applicant: Amano Enzyme Inc., Nagoya-shi Aichi 460-8630 (JP)
(72) Inventor: MATSUBARA, Hirotaka, Kakamigahara-shi, Gifu 509-0109 (JP)
(74) Representative: Müller-Boré & Partner Patentanwälte PartG mbB
(86) International application number: PCT/JP2022/043242
(87) International publication number: WO 2023/090461

(57) **Abstract**

This invention provides a novel protein-deamidating enzyme. This protein-deamidating enzyme includes a polypeptide that contains the amino acid sequence given by SEQ ID NO: 1 or a partial sequence thereof (the amino acid sequence of positions 1 to 221 in SEQ ID NO: 1, the amino acid sequence of positions 36 to 231 in SEQ ID NO: 1, or the amino acid sequence of positions 36 to 221 in SEQ ID NO: 1); a polypeptide that has a protein glutaminase activity and contains an amino acid sequence obtained by substituting, adding, inserting, or deleting one or a plurality of amino acids in the amino acid sequence given by SEQ ID NO: 1 or the partial sequences thereof; or a polypeptide that has a protein glutaminase activity and contains an amino acid sequence that has a 70% or higher sequence identity with the amino acid sequence given by SEQ ID NO: 1 or the partial sequences thereof.

## Description

### TECHNICAL FIELD

The present invention relates to a novel protein deamidase.

### BACKGROUND ART

The protein deamidase is an enzyme that catalyzes a deamidation reaction by acting on a protein that is a polymer. The protein deamidase deamidates a glutamine residue in a protein to generate a negatively charged carboxyl group, thus causing various characteristic changes to the protein. For example, an increase in hydration power and an increase in electrostatic repulsive force, which are caused by a decrease in the isoelectric point of the protein, reduce the interaction between proteins (that is, reduce associative properties), and thus increase the solubility and water dispersibility of the protein. In addition, the appearance of the internal hydrophobic region due to the change in the higher order structure of the protein imparts surface active properties to the protein, and thus improves the emulsifying power, emulsion stability, foaming property, and foaming stability of the protein. Since the protein deamidase can greatly change the properties of the protein in this way, the use of the protein has been dramatically increased. For this reason, the protein deamidase is very useful, and has attracted high interest in the art.

The first discovered protein deamidase is a protein glutaminase found from Chryseobacterium proteolyticum in 2000 (Non-Patent Document 1). However, protein glutaminase is also an extremely specialized enzyme that, despite its high utility and interest, has not been newly discovered over the ensuing 20 years. Protein glutaminase was discovered from Bacteroides helcogenes after about 20 years (Non-Patent Document 2), but as of November 2021, protein glutaminase industrially used is only protein glutaminase derived from C. proteolyticum.

### PRIOR ART DOCUMENT

### NON-PATENT DOCUMENTS

Non-Patent Document 1: A novel protein-deamidating enzyme from Chryseobacterium proteolyticum sp. nov., a newly isolated bacterium from soil. Applied and Environmental Microbiology 2000; 66(8): pp. 3337 to 43
Non-Patent Document 2: A novel protein glutaminase from Bacteroides helcogenes-characterization and comparison, Applied Microbiology and Biotechnology (2020) 104: pp. 187 to 199

### SUMMARY OF THE INVENTION

### PROBLEMS TO BE SOLVED BY THE INVENTION

Protein deamidase having a protein glutaminase activity has not been discovered for many years after the report of protein glutaminase derived from C. proteolyticum. Therefore, it has been established as general technical common knowledge of those skilled in the art that protein deamidase is a special enzyme that hardly exists in nature and cannot be obtained by ordinary screening. An example in which a protein deamidase having a protein glutaminase activity other than protein glutaminase derived from C. proteolyticum was found is only one example of protein glutaminase derived from B. helcogenes. The recognition of those skilled in the art on this enzyme still remains restricted to the technical common knowledge described above.

Under such circumstances, the present invention purposely aims to find a new protein deamidase.

### MEANS FOR SOLVING THE PROBLEM

The present inventors have intensively studied to find a new protein deamidase, but an enzyme having a protein deamidation activity has not been found at all from candidates having high sequence identity with known protein deamidases. However, as a result of screening by an original method, the present inventors have found that there is an enzyme having a protein deamidation activity although it is a protein having low sequence identity with known protein deamidases. In addition, the present inventors have also found that there is an enzyme having a very special sequence structure in which one of the amino acid residues constituting the active center of the enzyme is not a cysteine residue but a serine residue among the newly found enzymes having a protein deamidation activity. The present invention has been completed based on these findings.

That is, the present invention provides the inventions of the following aspects.

Item 1. A protein deamidase including a polypeptide as defined in any of (I) to (III) below:
(I) a polypeptide including (A) an amino acid sequence set forth in SEQ ID NO: 1, or (B) an amino acid sequence of positions 1 to 221 of SEQ ID NO: 1, (C) an amino acid sequence of positions 36 to 231 of SEQ ID NO: 1, or (D) an amino acid sequence of positions 36 to 221 of SEQ ID NO: 1;
(II) a polypeptide including an amino acid sequence in which one or several amino acids are substituted, added, inserted or deleted in the amino acid sequences (A) to (D), and having an activity of catalyzing a deamidation reaction of a glutamine residue of a protein; and
(III) a polypeptide including an amino acid sequence having a sequence identity of 70% or more to the amino acid sequences (A) to (D), and having an activity of catalyzing a deamidation reaction of a glutamine residue of a protein.

Item 2. A DNA encoding the protein deamidase according to item 1.

Item 3. An expression cassette or a recombinant vector including the DNA according to item 2.

Item 4. A transformant obtained by transforming a host using the expression cassette or recombinant vector according to item 3.

Item 5. A method for producing a protein deamidase, the method including the step of culturing the transformant according to item 4.

Item 6. An enzyme preparation including the protein deamidase according to item 1.

Item 7. The enzyme preparation according to item 6, which is a modifier for a food and drink, or a food and drink material, an industrial material, or a pharmaceutical material containing a protein.

Item 8. A method for producing a protein in which a glutamine residue is deamidated, the method including the step of allowing the protein deamidase according to item 1 to act on a protein.

Item 9. The method according to item 8, in which the protein is contained in a food and drink, or a food and drink material, an industrial material, or a pharmaceutical material.

Item 10. A method for producing a modified food and drink, or food and drink material, industrial material, or pharmaceutical material, the method including the step of allowing the protein deamidase according to item 1 to act on a food and drink, or a food and drink material, an industrial material, or a pharmaceutical material containing a protein.

### ADVANTAGES OF THE INVENTION

According to the present invention, a new protein deamidase is provided.

### EMBODIMENTS OF THE INVENTION

As used herein, the term "non-polar amino acid" includes alanine, valine, leucine, isoleucine, proline, methionine, phenylalanine, and tryptophan. The term "uncharged amino acid" includes glycine, serine, threonine, cysteine, tyrosine, asparagine, and glutamine. The term "acidic amino acid" includes aspartic acid and glutamic acid. The term "basic amino acid" includes lysine, arginine, and histidine.

### 1. Protein deamidase

The protein deamidase of the present invention is a protein deamidase derived from Chryseobacterium nematophagum and a protein deamidase having a sequence similar to that of the protein deamidase.

Specifically, the protein deamidase of the present invention includes a polypeptide as defined in any of (I) to (III) below:
(I) a polypeptide including (A) the amino acid sequence set forth in SEQ ID NO: 1 (hereinafter, also referred to as "amino acid sequence (A)"), or (B) the amino acid sequence of positions 1 to 221 of SEQ ID NO: 1 (hereinafter, also referred to as "amino acid sequence (B)"), (C) the amino acid sequence of positions 36 to 231 of SEQ ID NO: 1 (hereinafter, also referred to as "amino acid sequence (C)"), or (D) the amino acid sequence of positions 36 to 221 of SEQ ID NO: 1 (hereinafter, also referred to as "amino acid sequence (D)");
(II) a polypeptide including an amino acid sequence in which one or several amino acids are substituted, added, inserted or deleted in the amino acid sequences (A) to (D), and having an activity of catalyzing a deamidation reaction of a glutamine residue of a protein; and
(III) a polypeptide including an amino acid sequence having a sequence identity of 70% or more to the amino acid sequences (A) to (D), and having an activity of catalyzing a deamidation reaction of a glutamine residue of a protein.

Hereinafter, the protein deamidase including the polypeptides of (I) to (III) will be described in detail. In the following description, among the polypeptides of (I) to (III), a polypeptide including the above-described amino acid sequence (A) as a base is also referred to as "full-length polypeptide", and its sequence is also referred to as "full-length amino acid sequence", and a polypeptide including the above-described amino acid sequences (B) to (D) as bases is also referred to as "non-full-length polypeptide", and its sequence is also referred to as "non-full-length amino acid sequence".

### 1-1. Protein deamidase including polypeptide of (I)

The amino acid sequence (A), that is, SEQ ID NO: 1, is a full-length amino acid sequence of an example of a protein deamidase derived from Chryseobacterium nematophagum. The amino acid sequence (A) is a sequence that consists of 231 amino acid residues and includes a sequence estimated to be a pro-sequence and a C-terminal sequence.

The amino acid sequence (B), that is, the amino acid sequence of positions 1 to 221 of SEQ ID NO: 1 is an amino acid sequence in which the C-terminal sequence has been removed from the amino acid sequence (A).

The amino acid sequence (C), that is, the amino acid sequence of positions 36 to 231 of SEQ ID NO: 1 is an amino acid sequence in which the sequence estimated to be the pro-sequence has been removed from the amino acid sequence (A).

The amino acid sequence (D), that is, the amino acid sequence of positions 36 to 221 of SEQ ID NO: 1 is an amino acid sequence in which the sequence estimated to be the pro-sequence and the C-terminal sequence have been removed from the amino acid sequence (A).

In the amino acid sequences (A) to (D), the amino acid residue at position 64 constituting the active center in SEQ ID NO: 1 is a serine residue. In known protein deamidases that deamidate a glutamine residue of a protein, one of the amino acid residues constituting the active center is a cysteine residue. Therefore, the protein deamidase including a polypeptide including the amino acid sequences (A) to (D) has an extremely special sequence structure that is not present in known protein deamidases in that it contains a serine residue. Furthermore, the polypeptide of (I) is preferable in that the active center includes a serine residue, and thus excellent oxidation stability can be expected as compared with normal protein deamidases.

Preferred examples of the polypeptide of (I) include a polypeptide including the amino acid sequence set forth in SEQ ID NO: 1 (amino acid sequence (A)), a polypeptide including the amino acid sequence set forth in SEQ ID NO: 2 (amino acid sequence (B)), a polypeptide including the amino acid sequence (C), and a polypeptide including the amino acid sequence (D).

Other preferred examples of the polypeptide of (I) include a non-full-length polypeptide from which an N-terminal sequence including a pro-sequence and/or a C-terminal sequence has been removed, that is, a polypeptide including the amino acid sequence set forth in SEQ ID NO: 2 (amino acid sequence (B)), a polypeptide including the amino acid sequence (C), and a polypeptide including the amino acid sequence (D), from the viewpoint of, for example, improving the activity of catalyzing a deamidation reaction of a glutamine residue of a protein.

Specific examples of the polypeptide of (I) include a polypeptide including the amino acid sequence set forth in SEQ ID NO: 1 (amino acid sequence (A)) and a polypeptide including the amino acid sequence set forth in SEQ ID NO: 2 (amino acid sequence (B)).

### 1-2. Protein deamidase including polypeptides of (II) and (III)

The polypeptides of (II) and (III) are protein deamidases that are similar in sequence to the polypeptide of (I) and have the amino acid sequence of the polypeptide of (I) as a basic skeleton.

The modification of the amino acid to be introduced into the polypeptide of (II) may include only one modification (for example, substitution) from among substitution, addition, insertion, and deletion, or may include two or more modifications (for example, substitution and insertion). In the polypeptide of (II), the number of amino acids to be substituted, added, inserted or deleted may be 1 or more or several, and is, for example, 1 to 10, preferably 1 to 8, 1 to 6, 1 to 5, or 1 to 4, more preferably 1 to 3, and particularly preferably 1 or 2, or 1.

In the polypeptide of (III), the sequence identity may be 70% or more, but is preferably 80% or more, 85% or more, 90% or more, still more preferably 95% or more, 97% or more, 98% or more, particularly preferably 99% or more, 99.5% or more.

Here, in the polypeptide of (III), the sequence identity to the amino acid sequences (A) to (D) is a sequence identity calculated as compared to each of the amino acid sequences (A) to (D). In addition, the "sequence identity" indicates a value of identity of an amino acid sequence obtained by a bl2seq program (Tatiana A. Tatsusova, Thomas L. Madden, FEMS Microbiol. Lett., Vol. 174, pp. 247 to 250, 1999) of BLAST PACKAGE [sgi32 bit edition, Version 2.0.12; available from National Center for Biotechnology Information (NCBI)]. The parameters may be set to Gap insertion Cost value: 11 and Gap extension Cost value: 1.

In the polypeptides of (II) and (III), the serine residue at position 64, the histidine residue at position 105, and the aspartic acid residue at position 124 in the amino acid sequence set forth in SEQ ID NO: 1 contribute to the activity. Among these amino acid residues, it is desirable that no substitution or deletion is introduced into the serine residue at position 64 except for substitution with an amino acid residue other than the cysteine residue, and it is desirable that no substitution or deletion is introduced into the histidine residue at position 105 and the aspartic acid residue at position 124.

When an amino acid substitution is introduced into the polypeptides of (II) and (III), conservative substitution is mentioned as an aspect of the amino acid substitution. That is, examples of the amino acid substitution to be introduced into the amino acid sequences (A) to (D) in the polypeptides of (II) and (III) include substitution with another non-polar amino acid if the amino acid before substitution is a non-polar amino acid, substitution with another uncharged amino acid if the amino acid before substitution is an uncharged amino acid, substitution with another acidic amino acid if the amino acid before substitution is an acidic amino acid, and substitution with another basic amino acid if the amino acid before substitution is a basic amino acid.

When an amino acid addition is introduced into the polypeptides of (II) and (III), examples of an aspect of the amino acid addition include addition of a methionine residue to the N-terminal, and addition of a purification tag (for example, a binding oligopeptide such as oligohistidine).

The polypeptides of (II) and (III) include not only polypeptides obtained by artificial mutation but also polypeptides generated by naturally occurring mutations (mutants or variants) based on individual differences or species differences in an organism from which polypeptides are derived.

In the polypeptides of (II) and (III), the "activity of catalyzing a deamidation reaction of a glutamine residue of a protein" (hereinafter, also described as "protein glutaminase activity") can be confirmed by detection of a product associated with deamidation of the glutamine residue. For example, the protein glutaminase activity can be confirmed by causing a protein deamidase to act using two or more peptides (for example, N-benzyloxycarbonyl-L-glutaminylglycine (Z-Gln-Gly)) containing a glutamine residue and not containing an asparagine residue as a substrate, and detecting a substrate in which a glutamine residue has been converted to a glutamic acid residue or released ammonia as a product. The protein glutaminase activity value can be quantitatively calculated based on the amount of the product, preferably the amount of released ammonia.

Among the polypeptides of (II) and (III), a polypeptide in which the serine residue at position 64 in the amino acid sequence set forth in SEQ ID NO: 1 is maintained is particularly preferable in that excellent oxidation stability can be expected as compared with that of normal protein deamidases. This is because the active center of such a polypeptide includes a serine residue.

Other preferred examples of the polypeptides of (II) and (III) include a polypeptide that includes an amino acid sequence obtained by substituting, adding, inserting, or deleting one or several amino acids in the amino acid sequences (A) to (D) and has an activity of catalyzing a deamidation reaction of a glutamine residue of a protein, and a polypeptide that includes an amino acid sequence having a sequence identity of 70% or more (preferably 80% or more, 85% or more, 90% or more, still more preferably 95% or more, 97% or more, 98% or more, particularly preferably 99% or more, 99.5% or more) to the amino acid sequences (A) to (D) and has an activity of catalyzing a deamidation reaction of a glutamine residue of a protein.

Further preferred other examples of the polypeptides of (II) and (III) include a non-full-length polypeptide from which an N-terminal sequence including a pro-sequence and/or a C-terminal sequence has been removed, that is, a polypeptide that includes an amino acid sequence obtained by substituting, adding, inserting or deleting one or several amino acids in (B) to (D) and has an activity of catalyzing a deamidation reaction of a glutamine residue of a protein, and a polypeptide that includes an amino acid sequence having a sequence identity of 70% or more (preferably 80% or more, 85% or more, 90% or more, still more preferably 95% or more, 97% or more, 98% or more, particularly preferably 99% or more, 99.5% or more) to the amino acid sequences (B) to (D) and has an activity of catalyzing a deamidation reaction of a glutamine residue of a protein, from the viewpoint of, for example, improving a protein glutaminase activity.

Specific examples of the polypeptides of (II) and (III) include a polypeptide including the amino acid sequence set forth in SEQ ID NO: 3 (a sequence in which a methionine residue is added to the N-terminal in the amino acid sequence (D)), a polypeptide including the amino acid sequence set forth in SEQ ID NO: 4 (a sequence in which position 64 of SEQ ID NO: 1 is substituted with a cysteine residue in the amino acid sequence (A)), a polypeptide including the amino acid sequence set forth in SEQ ID NO: 5 (a sequence in which position 64 of SEQ ID NO: 1 is substituted with a cysteine residue in the amino acid sequence (B)), and a polypeptide including the amino acid sequence set forth in SEQ ID NO: 6 (a sequence in which a methionine residue is added at the N-terminal in the amino acid sequence (D) and position 64 of SEQ ID NO: 1 is substituted with a cysteine residue).

Among the polypeptides of (I) to (III), for example, a polypeptide that includes an amino acid sequence having a sequence identity of 90% or more (more preferably 95% or more, still more preferably 97% or more, still even more preferably 98% or more, particularly preferably 99% or more, 99.5% or more) to any of the amino acid sequences of SEQ ID NO: 1 to 3 and has an activity of catalyzing a deamidation reaction of a glutamine residue of a protein is particularly preferred.

### 2. DNA

The DNA encoding the protein deamidase of the present invention (hereinafter, may be referred to as "DNA of the present invention") can be appropriately prepared and designed by those skilled in the art according to the amino acid sequence of the protein deamidase of the present invention.

The base sequence of the DNA of the present invention can be appropriately designed by those skilled in the art according to the amino acid sequence described in the protein deamidase of the present invention.

Examples of the sequence of the DNA of the present invention include the base sequence set forth in SEQ ID NO: 13. SEQ ID NO: 13 is a sequence encoding the protein deamidase set forth in SEQ ID NO: 1 derived from Chryseobacterium nematophagum.

The DNA of the present invention is preferably one in which the codon usage frequency is optimized for the host. For example, when Escherichia coli is used as a host, DNA in which the codon usage frequency is optimized for Escherichia coli is suitable.

Preferred examples of the DNA of the present invention include DNAs as defined in any one of the following (i) to (iii):

(i) a DNA including (a) the base sequence set forth in SEQ ID NO: 7 (hereinafter, also described as "base sequence (a)"), or (b) the base sequence of positions 1 to 663 of SEQ ID NO: 7 (hereinafter, also described as "base sequence (b)"), (c) the base sequence of positions 106 to 696 of SEQ ID NO: 7 (hereinafter, also described as "base sequence (c)"), or (d) the base sequence of positions 106 to 663 of SEQ ID NO: 7 (hereinafter, also described as "base sequence (d)");
(ii) a DNA including a base sequence of a DNA that hybridizes with a DNA including a base sequence complementary to the base sequences (a) to (d) under a stringent condition; and
(iii) a DNA including a base sequence having a homology of 70% or more to the base sequences (a) to (d).

Hereinafter, the DNAs of (i) to (iii) will be described in detail.

### 2-1. DNA of (i)

The base sequence (a), that is, SEQ ID NO: 7 is a sequence that consists of 696 bases encoding SEQ ID NO: 1, which is a full-length amino acid sequence of a protein deamidase derived from Chryseobacterium nematophagum, and that is obtained by optimizing the codon usage frequency of the base sequence set forth in SEQ ID NO: 13 for Escherichia coli.

The base sequence (b), that is, the base sequence of positions 1 to 663 of SEQ ID NO: 7 is a base sequence in which the sequence encoding the sequence estimated to be the C-terminal sequence has been removed from the base sequence (a).

The base sequence (c), that is, the base sequence of positions 106 to 696 of SEQ ID NO: 7 is a base sequence in which the sequence encoding the sequence estimated to be the pro-sequence has been removed from the base sequence (a).

In the base sequence (d), that is, the base sequence of positions 106 to 663 of SEQ ID NO: 7 is a base sequence in which the sequence encoding the sequence estimated to be the C-terminal sequence and the sequence encoding the sequence estimated to be the pro-sequence have been removed from the base sequence (a).

Preferred examples of the DNA of (i) include a DNA including the base sequence set forth in SEQ ID NO: 7 (base sequence (a)), a DNA including the base sequence (b), a DNA including the base sequence (c), and a DNA including the base sequence (d).

Other preferred examples of the DNA of (i) include a sequence encoding a non-full-length polypeptide, that is, a DNA including the base sequence (b), a DNA including the base sequence (c), and a DNA including the base sequence (d), from the viewpoint of, for example, improving an activity of catalyzing a deamidation reaction of a glutamine residue of a protein.

### 2-2. DNAs of (ii) and (iii)

The DNAs of (ii) and (iii) are sequence-similar DNAs including the base sequence of the DNA of (i) as a basic skeleton.

In the DNA of (ii), the expression "under a stringent condition" refers to a condition of incubating for 4 hours to overnight at 50°C to 65°C in 6 × SSC (1 × SSC is 0.15 M NaCl, 0.015 M sodium citrate, pH 7.0) containing 0.5% SDS, 5 × Denhartz's [0.1% bovine serum albumin (BSA), 0.1% polyvinylpyrrolidone, 0.1% Ficoll 400] and 100 µg/ml salmon sperm DNA.

Hybridization under a stringent condition is specifically performed by the following method. That is, a nylon membrane on which a DNA library or a cDNA library is immobilized is prepared, and the nylon membrane is blocked at 65°C in a prehybridization solution containing 6 × SSC, 0.5% SDS, 5 × Denhartz's, and 100 µg/ml salmon sperm DNA. Each probe labeled with ³²P is then added, and this is incubated overnight at 65°C. This nylon membrane is washed in 6 × SSC at room temperature for 10 minutes, in 2 × SSC containing 0.1% SDS at room temperature for 10 minutes, in 0.2 × SSC containing 0.1% SDS at 45°C for 30 minutes, and then autoradiography is performed, and a DNA which specifically hybridizes with the probe can be detected.

In the DNA of (iii), the homology may be 70% or more, and is preferably 80% or more, 90% or more, more preferably 95% or more, 97% or more, 98% or more, and particularly preferably 99% or more, 99.5% or more.

Here, the "homology" of the base sequence indicates a value of identity obtained by a bl2seq program (Tatiana A. Tatsusova, Thomas L. Madden, FEMS Microbiol. Lett., Vol. 174, pp. 247 to 250, 1999) of BLAST PACKAGE [sgi32 bit edition, Version 2.0.12; available from the National Center for Biotechnology Information (NCBI)]. The parameters may be set to Gap insertion Cost value: 11 and Gap extension Cost value: 1.

In the examples of the DNAs of (ii) and (iii), at least one of base sequences encoding a start codon, a stop codon, and a purification tag (for example, a binding oligopeptide such as oligohistidine) may be further added to the DNA of (i).

Preferred examples of the DNAs of (ii) and (iii) include a DNA including a base sequence of a DNA that hybridizes with a DNA having a base sequence complementary to the base sequences (a) to (d) under a stringent condition, and a DNA including a base sequence having a homology of 70% or more with the base sequences (a) to (d).

Other preferred examples of the DNAs of (ii) and (iii) include a sequence encoding a non-full-length polypeptide, that is, a DNA including a base sequence of a DNA that hybridizes with a DNA having a base sequence complementary to the base sequences (b) to (d) under a stringent condition, and a DNA including a base sequence having a homology of 70% or more to the base sequences (b) to (d), from the viewpoint of, for example, improving an activity of catalyzing a deamidation reaction of a glutamine residue of a protein.

Specific examples of the DNAs of (ii) and (iii) include a DNA including the base sequence set forth in SEQ ID NO: 8 (a sequence in which a stop codon is added in the base sequence (b)), a DNA including the base sequence set forth in SEQ ID NO: 9 (a sequence in which a start codon and a stop codon are added in the base sequence (d)), a DNA including the base sequence shown in SEQ ID NO: 10 (a sequence in which positions 190 to 192 of SEQ ID NO. 7 in the base sequence (a) are substituted with a sequence encoding a cysteine residue), a DNA including the base sequence shown in SEQ ID NO: 11 (a sequence in which a stop codon is added in the base sequence (b) and positions 190 to 192 of SEQ ID NO: 7 are substituted with a sequence encoding a cysteine residue), and a DNA including the base sequence shown in SEQ ID NO: 12 (a sequence in which a start codon and a stop codon are added in the base sequence (d), and positions 190 to 192 of SEQ ID NO: 7 are substituted with a sequence encoding a cysteine residue).

### 2-3. Preparation of DNA

Examples of the method for obtaining the DNA of the present invention include the following method based on hybridization.

First, a DNA obtained from an appropriate gene source is connected to a plasmid or a phage vector according to a standard method to prepare a DNA library. A transformant obtained by introducing this library into an appropriate host is cultured on a plate, the grown colonies or plaques are transferred to a nitrocellulose or nylon membrane, and the DNA is fixed to the membrane after denaturation treatment. This membrane is incubated in a solution having the above composition containing a probe labeled with ³²P or the like in advance under the above stringent condition to perform hybridization. As the probe, a polynucleotide encoding the polypeptide of any one of (I) to (III) can be used.

After completion of hybridization, the non-specifically adsorbed probe is washed away, and a clone hybridized with the probe is identified by autoradiography or the like. This operation is repeated until the hybridized clone can be isolated. Finally, a gene encoding a protein having a desired enzyme activity is selected from the obtained clones. The gene can be isolated by a known polynucleotide extraction method such as an alkaline method.

The DNA of the present invention can also be isolated from Chryseobacterium nematophagum. For example, using genomic DNA derived from Chryseobacterium nematophagum as a template, a desired DNA can be isolated from the genome of the microorganism by PCR or hybridization using a primer or a probe designed in consideration of the degeneracy of the gene from known amino acid sequence information or a primer or a probe designed based on known base sequence information.

The DNA of the present invention includes various types of DNAs derived from codon degeneracy. Artificially producing various types of DNAs encoding the same amino acid sequence can be easily performed using known genetic engineering techniques. For example, in the production of a genetically engineered protein, when the usage frequency of the codon used on an original gene encoding a desired protein is low in the host, the expression level of the protein may be low. In such a case, high expression of the target protein can be achieved by optimizing the codon usage frequency for the host without changing the encoded amino acid sequence.

As an index representing the codon usage frequency, the total host optimal codon usage frequency of each codon may be adopted. The optimal codon is defined as a codon having the highest usage frequency among codons corresponding to the same amino acid. The codon usage frequency is not particularly limited as long as it is optimized for a host, and examples of the optimal codon of Escherichia coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), G: glycine (ggc).

As a method for artificially modifying an amino acid sequence by introducing mutation into a gene, a mutation introduction kit using a known method such as the Kunkel method or the Gapped duplex method, and site-directed mutagenesis can be used. Examples of the kit include QuikChangeTM Site-Directed Mutagenesis Kit (Stratagene), GeneArtTM Site-Directed Mutagenesis PLUS System (Invitrogen Corporation), and TaKaRa Site-Directed Mutagenesis System (Mutan-K, Mutan-Super Express Km, PrimeSTAR Mutagenesis Basal Kit, etc.: Takara Bio Inc.).

The base sequence of DNA can be confirmed by sequencing using a common method. For example, sequencing can be carried out by the dideoxynucleotide chain termination method (Sanger et al. (1977) Proc. Natl. Acad. Sci. USA 74:5463). In addition, the sequence can be analyzed using an appropriate DNA sequencer.

Whether or not the obtained DNA is a DNA encoding a desired protein deamidase can be confirmed by comparing the determined base sequence with the base sequences (a) to (d). Alternatively, the confirmation can be carried out by comparing an amino acid sequence estimated from the determined base sequence with the amino acid sequences (A) to (D).

### 3. Expression cassette or recombinant vector

The expression cassette or recombinant vector (hereinafter, also referred to as "expression cassette of the present invention" or "recombinant vector of the present invention") containing the DNA encoding the protein deamidase of the present invention includes the DNA encoding the protein deamidase of the present invention.

The expression cassette or recombinant vector of the present invention can be obtained by linking a promoter and a terminator to the DNA of the present invention, or by inserting the expression cassette or the DNA of the present invention into an expression vector.

The expression cassette or recombinant vector of the present invention may further include, as a control factor, a transcription element such as an enhancer, a CCAAT box, a TATA box, or an SPI site, as necessary, in addition to a promoter and a terminator. These control factors may be operably linked to the DNA of the present invention. The expression "operably linking" means that various control factors that regulate the DNA of the present invention and the DNA of the present invention are linked in a state of being operable in a host cell.

When a non-full-length polypeptide is expressed, the expression cassette of the present invention or the recombinant vector of the present invention may be designed to directly express the non-full-length polypeptide, or as described below, may be designed so that after the full-length polypeptide including the protease recognition sequence is once expressed, the terminal sequence thereof can be cleaved by a protease.

When the expression cassette of the present invention or the recombinant vector of the present invention is designed so that after a full-length polypeptide including a protease recognition sequence is once expressed, the terminal sequence can be cleaved by a protease, the expression cassette or the recombinant vector of the present invention can be configured to include a base sequence encoding a protease recognition sequence and a base sequence encoding an N-terminal sequence and/or a C-terminal sequence in combination. Specifically, in addition to the base sequence encoding the non-full-length polypeptide, the expression cassette or the recombinant vector of the present invention can be configured to include a base sequence encoding a recognition sequence for a specific protease between a base sequence encoding an N-terminal sequence including a pre-sequence and/or a pro-sequence and a base sequence encoding a non-full-length polypeptide and the like, and/or between a base sequence encoding a non-full-length polypeptide and the like and a base sequence encoding a C-terminal sequence. In this case, after a full-length polypeptide is expressed, the N-terminal sequence and/or the C-terminal sequence is specifically removed using the specific protease, whereby a protein deamidase including a non-full-length polypeptide can be obtained.

As the expression vector, those constructed for genetic recombination from phage, plasmid, or virus capable of autonomously growing in a host are suitable. Such expression vectors are known, and those skilled in the art can appropriately select and use an appropriate combination with a host cell. Examples of the vector in the case of using a microorganism as a host include pBluescript (pBS) II SK (-) (manufactured by Stratagene), a pSTV-based vector (manufactured by Takara Bio Inc.), a pUC-based vector (manufactured by Takara Bio Inc.), a pET-based vector (manufactured by Sigma-Aldrich Japan), a pGEX-based vector (manufactured by Global Life Sciences Technologies Japan K.K. (Cytiva)), a pCold-based vector (manufactured by Takara Bio Inc.), pHY300PLK (manufactured by Takara Bio Inc.), pUB110 (Mckenzie, T. et al., 1986, Plasmid 15(2), pp. 93 to 103), pBR322 (manufactured by Takara Bio Inc.), pRS403 (manufactured by Stratagene), and pMW218/219 (manufactured by Nippon Gene Co., Ltd.). Examples of the vector in the case of using algae or microalgae as a host include pUC19 (manufactured by Takara Bio Inc.), P66 (Chlamydomonas Center), P-322 (Chlamydomonas Center), pPha-T1 (see, Yangmin Gong, et al., Journal of Basic Microbiology, 2011, vol. 51, p. 666 to 672), and pJET1 (manufactured by Thermo Fisher Scientific Inc.). Examples of the vector in the case of using a plant cell as a host include a pRI-based vector (manufactured by Takara Bio Inc.), a pBI-based vector (manufactured by Clontech Laboratories, Inc.), and an IN3-based vector (manufactured by Inplanta Innovations Inc.).

### 4. Transformant

A transformant (hereinafter, may be referred to as "transformant of the present invention") is obtained by transforming a host using the expression cassette or recombinant vector of the present invention.

The host used for the production of the transformant is not particularly limited as long as a gene can be introduced in the host, and the host can autonomously grow and express the trait of the gene of the present invention. Suitable examples of the host include microorganisms including bacteria belonging to the genus Escherichia such as Escherichia coli, the genus Bacillus such as Bacillus subtilis, and the genus Pseudomonas such as Pseudomonas putida; actinomycetes; yeasts; and filamentous fungi, but the host may also be animal cells, insect cells, plants, and the like. Among them, Escherichia coli is particularly preferable.

The host used for the production of the transformant may be Chryseobacterium nematophagum which is a derived bacterium of the protein deamidase of the present invention.

The transformant of the present invention can be obtained by introducing the expression cassette or recombinant vector of the present invention into a host. The site where the DNA of the present invention is introduced is not particularly limited as long as a desired gene can be expressed, and may be on the plasmid or on the genome. Specific examples of the method for introducing the expression cassette of the present invention or the recombinant vector of the present invention include a recombinant vector method and a genome editing method.

Conditions for introducing the expression cassette or recombinant vector of the present invention into the host may be appropriately set according to the type of the host and the like. Examples of the method in the case of using a microorganism as a host include a method using a competent cell by calcium ion treatment, an electroporation method, a spheroplast method, and a lithium acetate method. Examples of the method in the case of using an animal cell as a host include an electroporation method, a calcium phosphate method, and a lipofection method. Examples of the method in the case of using an insect cell as a host include a calcium phosphate method, a lipofection method, and an electroporation method. Examples of the method in the case of using a plant cell as a host include an electroporation method, an Agrobacterium method, a particle gun method, and a PEG method.

### 5. Method for producing protein deamidase

The present invention also provides a method for producing the protein deamidase described above. The protein deamidase can be produced by culturing the transformant of the present invention. The protein deamidase described above can also be produced by culturing Chryseobacterium nematophagum itself (which is not transformed), which is a derived bacterium or a derived cell.

The culture conditions of the transformant or the derived bacterium or derived cell of the present invention may be appropriately set in consideration of the nutritional physiological properties of the host or the derived bacterium or derived cell, and liquid culture is preferable. In the case of industrial production, aeration stirring culture is preferable.

The transformant or the derived bacterium or derived cell of the present invention is cultured, and the culture supernatant or the cultured bacterial cell or cultured cell is recovered by a method such as centrifugation of the culture solution. When the protein deamidase of the present invention is accumulated in the cultured bacterial cell or cultured cell, the bacterial cell or cell is treated with a mechanical method such as ultrasonic waves or French press or a lytic enzyme such as lysozyme, and if necessary, solubilized using an enzyme such as protease or a surfactant such as sodium dodecyl sulfate (SDS), whereby a water-soluble fraction containing the protein deamidase of the present invention can be obtained.

In addition, by selecting an appropriate expression vector and host, the expressed protein deamidase of the present invention may be secreted into the culture solution.

In one embodiment of the method for producing a protein deamidase, a full-length protein can be directly expressed by selecting, as an expression cassette or recombinant vector used for producing a transformant, an expression cassette or recombinant vector configured to be capable of expressing a full-length polypeptide. In another embodiment, a non-full-length protein can be directly expressed by selecting, as an expression cassette or recombinant vector used for producing a transformant, an expression cassette or recombinant vector configured to be capable of expressing a non-full-length polypeptide.

Further, in the above one embodiment, there are a case of selecting, as an expression cassette or recombinant vector used for producing the transformant, an expression cassette or recombinant vector configured to include a base sequence encoding a full-length polypeptide, and a case of selecting an expression cassette or recombinant vector configured to include a base sequence encoding a non-full-length polypeptide in combination with a base sequence encoding a protease recognition sequence and a base sequence encoding an N-terminal sequence and/or a C-terminal sequence. The method for producing a protein deamidase of the present invention in these cases may further include a step of removing the N-terminal sequence and/or the C-terminal sequence (step of obtaining a non-full-length polypeptide) for the purpose of obtaining a non-full-length polypeptide from a full-length polypeptide that has been once expressed as described below.

In an example of the step of obtaining a non-full-length polypeptide, an expression cassette or recombinant vector configured to include a base sequence encoding a full-length polypeptide is selected as an expression cassette or recombinant vector used for producing a transformant, and removal of the N-terminal sequence can be performed, for example, by treating a protein deamidase with a processing enzyme. Examples of the processing enzyme include proteases. Specific examples of the protease include serine proteases such as subtilisin, chymotrypsin, and trypsin; cysteine proteases such as papain, bromelain, caspase, and calpain; acidic proteases such as pepsin and cathepsin; and metal proteases such as thermolysin.

As another example of the step of obtaining a non-full-length polypeptide, an expression cassette or recombinant vector configured to include a base sequence encoding a protease recognition sequence and a base sequence encoding an N-terminal sequence and/or a C-terminal sequence in combination with a base sequence encoding a non-full-length polypeptide is selected as an expression cassette or recombinant vector used for producing a transformant, then a full-length polypeptide including a recognition sequence of a specific protease is expressed between the base sequence encoding the N-terminal sequence and the base sequence encoding the non-full-length polypeptide and/or between the base sequence encoding the non-full-length polypeptide and the base sequence encoding the C-terminal sequence, after which the N-terminal sequence and/or the C-terminal sequence can be specifically removed using the specific protease.

The culture solution, water-soluble fraction, or protease-treated product containing the protein deamidase of the present invention obtained as described above may be subjected to a purification treatment as it is, or may be subjected to a purification treatment after the protein deamidase of the present invention in the culture solution, the water-soluble fraction, or the protease-treated product is concentrated.

The concentration can be performed by, for example, concentration under reduced pressure, membrane concentration, salting-out treatment, and fractionation precipitation with a hydrophilic organic solvent (for example, methanol, ethanol, and acetone).

The purification treatment of the protein deamidase of the present invention can be performed, for example, by appropriately combining methods such as gel filtration, hydrophobic chromatography, ion exchange chromatography, and affinity chromatography.

The protein deamidase of the present invention thus purified may be powdered by lyophilization, vacuum drying, spray drying, or the like as necessary.

### 6. Enzyme preparation

The protein deamidase of the present invention can be provided in the form of an enzyme agent. Therefore, the present invention also provides an enzyme agent containing the protein deamidase of the present invention.

The content of the protein deamidase of the present invention in the enzyme agent of the present invention is not particularly limited, and can be appropriately set within a range in which the protein glutaminase activity is exhibited.

The enzyme agent of the present invention may contain, in addition to the protein deamidase of the present invention, other components to the extent that the effect of the present invention is not affected. Examples of the other components include enzymes other than the protein deamidase of the present invention, additives, and culture residues generated by the above production method.

The other enzymes can be appropriately determined according to its use, and examples thereof include amylases (α-amylase, β-amylase, glucoamylase), glucosidase (α-glucosidase, β-glucosidase), galactosidases (α-galactosidase, β-galactosidase), proteases (acidic protease, neutral protease, alkaline protease), peptidases (leucine peptidase, aminopeptidase), lipase, esterase, cellulase, phosphatase (acidic phosphatase, alkaline phosphatase), nuclease, deaminase, oxidase, dehydrogenase, glutaminase, pectinase, catalase, dextranase, transglutaminase, protein deamidases (other than the protein deamidase of the present invention), and pullulanase. These other enzymes may be contained alone or in combination of two or more thereof.

The additive can be appropriately determined according to the use of the polypeptide of the present invention and the formulation form of the enzyme agent. Examples of the additive include excipients, buffers, suspensions, stabilizers, preservatives, antiseptics, and saline. Examples of the excipient include starch, dextrin, maltose, trehalose, lactose, D-glucose, sorbitol, D-mannitol, sucrose, and glycerol. Examples of the buffer include phosphate, citrate, and acetate. Examples of the stabilizer include propylene glycol and ascorbic acid. Examples of the preservative include benzoates (alkali metal salts such as potassium salts and sodium salts), sorbates (alkali metal salts such as potassium salts and sodium salts), phenol, benzalkonium chloride, benzyl alcohol, chlorobutanol, and methylparaben. Examples of the antiseptic include ethanol, benzalkonium chloride, parahydroxybenzoic acid, and chlorobutanol. These additives may be contained alone or in combination of two or more thereof.

Examples of the culture residue include components derived from a medium, contaminant proteins, bacterial cell components, and cell components.

The formulation form of the enzyme agent of the present invention is not particularly limited, and examples thereof include liquid and solid forms (powder, granules, etc.). The enzyme agent of these formulation forms can be prepared by a generally known method.

Specific examples of the enzyme agent of the present invention include a food and drink containing a protein or a modifier for a raw material thereof. Other specific examples of the enzyme agent of the present invention include reagents for protein analysis and/or research, such as a reagent for determining the amide group content of a protein and a reagent for solubilizing a protein.

### 7. Utilization of protein deamidase

The protein deamidase of the present invention can be used for any application utilizing the deamidation action of a glutamine residue in a protein. Therefore, the present invention also provides a method for producing a protein in which a glutamine residue is deamidated, the method including the step of allowing the protein deamidase of the present invention to act on a protein.

The protein (substrate protein) deamidated by the protein deamidase is not particularly limited as long as it is a protein containing a glutamine residue. The length of the substrate protein is not particularly limited as long as it is two residues (dipeptide) or more, and the substrate protein includes not only a protein but also a peptide. The substrate protein includes both natural products and artificial products. When the substrate protein is a natural product, the origin of the substrate protein may be either an animal or a plant. Furthermore, the substrate protein may be a protein contained in a food and drink or a raw material thereof, or may be a protein used as an analysis target or a research target.

The protein deamidase can generate a negatively charged carboxyl group by deamidation of a glutamine residue in a protein. Therefore, the protein deamidase can change (modify) the properties of the protein by an action similar to the known action of known protein deamidases, such as increasing the solubility and water dispersibility of the protein or improving the emulsifying power, emulsion stability, foaming property, and foaming stability of the protein. Therefore, the protein deamidase is particularly useful when a protein contained in a food and drink or a food and drink material (food and drink raw material) is used as a substrate. Furthermore, the protein deamidase is not limited to a food and drink or a food and drink material, and is also useful when a protein contained in an industrial material used in the fiber field, the cosmetic field, or the like, and a protein contained in a pharmaceutical material are used as a substrate. Therefore, the present invention also provides a method for producing a modified food and drink or food and drink material, industrial material, or pharmaceutical material, the method including the step of allowing the protein deamidase of the present invention to act on a food and drink or a food and drink material, an industrial material, or a pharmaceutical material containing a protein.

In the method for producing a protein in which a glutamine residue is deamidated and the method for producing a modified food and drink or food and drink material, industrial material, or pharmaceutical material of the present invention, the reaction conditions (amount of enzyme, reaction time, reaction temperature, reaction pH, etc.) between the protein deamidase of the present invention and the substrate protein are not particularly limited as long as deamidation is achieved to a desired degree. Those skilled in the art can appropriately set the reaction conditions according to various conditions such as the type and/or purity of the protein deamidase of the present invention, the type and/or concentration of the substrate protein, and/or the desired degree of deamidation.

### EXAMPLE

Hereinafter, the present invention will be specifically described with reference to Examples, but the present invention is not to be construed as being limited to the following Examples.

### Test Example

The protein deamidase was prepared by the following procedure. Each of the base sequences of SEQ ID NO: 7 to 12 was cloned into pET21a to produce a plasmid. The obtained plasmid was introduced into E. coli BL21 (DE3) to obtain a transformant. The transformant was inoculated into a TB + Amp medium or an Over Night Expression TB (OETB) (manufactured by Sigma-Aldrich Japan) + Amp medium, and cultured at 37°C overnight under aeration and stirring using a jar. When the TB + Amp medium was used, IPTG was added to a final concentration of 0.1 mM 6 hours after the start of culture. After the bacterial cells in the resulting culture solution were collected, the bacterial cells were dissolved with lysozyme to collect a soluble fraction (protein deamidase fraction).

The protein glutaminase activity was evaluated for the obtained soluble fraction (protein deamidase fraction) by the following procedure. 10 µL of the soluble fraction was mixed with 130 µL of a substrate solution (5 mM Z-Gln-Gly, 0.0023% Triton X-100, 0.2 M monopotassium disodium phosphate, pH 6.5), and the solution was then reacted overnight at 37°C. To the reaction solution, 10 µL of hippuric acid (6.25 mM hippuric acid, 0.2 M monopotassium disodium phosphate, pH 6.5) as an internal standard material was added. After mixing, the sample filtered by MF was subjected to HPLC analysis under the following conditions to evaluate the presence or absence of the protein glutaminase activity according to the presence or absence of the peak of Z-Glu-Gly (product). The results are shown in Table 1.

### (HPLC conditions)

Column: ZORBAX SB-C18 2.1 × 50 mm, 1.8 µm
Solvent A: 0.1% TFA/H₂O Solvent B: 0.1% TFA/acetonitrile
Program: 0-0.3 min_B: 10%
0.3-2.0 min_B: 10-25%
2.0-2.2 min_B: 25%
2.2-3.0 min_B: 100%
Injection volume: 2 µL
Flow rate: 1.0 mL/min
Detection: UV 205 nm

**[Table 1]**

| | Base sequence | Amino acid sequence | Outline | Protein glutaminase activity |
|---|---|---|---|---|
| Comparative Example 1 | - | - | No enzyme | Absent |
| Example 1 | SEQ ID NO: 7 | SEQ ID NO: 1 | Full length | Present |
| Example 2 | SEQ ID NO: 8 | SEQ ID NO: 2 | C-terminal sequence removed | Present |
| Example 3 | SEQ ID NO: 9 | SEQ ID NO: 3 | C-terminal and N-terminal sequence-removed, and methionine-added | Present |
| Example 4 | SEQ ID NO: 10 | SEQ ID NO: 4 | S64C, full length | Present |
| Example 5 | SEQ ID NO: 11 | SEQ ID NO: 5 | S64C, C-terminal sequence removed | Present |
| Example 6 | SEQ ID NO: 12 | SEQ ID NO: 6 | S64C, C-terminal and N-terminal sequence-removed, and methionine-added | Present |

As shown in Table 1, it was confirmed that in all the prepared enzymes (Examples 1 to 6), the appearance of the peak of the product generated by deamidation of the glutamine residue was confirmed. That is, it was recognized that all the prepared enzymes were protein deamidases having a protein glutaminase activity. In general, existing protein glutaminases lose their activities when a cysteine residue at a catalytic site thereof is substituted with a serine residue. Therefore, it was an unexpected result that the enzyme has a protein glutaminase activity while having a serine residue at a catalytic site. In addition, it was also an unexpected result that the enzyme had a protein glutaminase activity even when a serine residue was substituted with a cysteine residue (Examples 4 to 6).

### (Sequence listing free text)

SEQ ID NO: 1 is the amino acid sequence of Chryseobacterium nematophagum protein deamidase.
SEQ ID NO: 2 represents a C-terminal sequence-removed sequence of SEQ ID NO: 1.
SEQ ID NO: 3 represents a C-terminal and N-terminal sequence-removed and methionine-added sequence of SEQ ID NO: 1.
SEQ ID NO: 4 represents an S64C-substituted sequence of SEQ ID NO: 1.
SEQ ID NO: 5 represents an S64C-substituted and C-terminal sequence-removed sequence of SEQ ID NO: 1.
SEQ ID NO: 6 represents an S64C-substituted, C-terminal and N-terminal sequence-removed, and methionine-added sequence of SEQ ID NO: 1.
SEQ ID NO: 7 is a base sequence that encodes SEQ ID NO: 1 and is codon optimized for E. coli.
SEQ ID NO: 8 is a base sequence that encodes SEQ ID NO: 2 and is codon optimized for E. coli.
SEQ ID NO: 9 is a base sequence that encodes SEQ ID NO: 3 and is codon optimized for E. coli.
SEQ ID NO: 10 is a base sequence that encodes SEQ ID NO: 4 and is codon optimized for E. coli.
SEQ ID NO: 11 is a base sequence that encodes SEQ ID NO: 5 and is codon optimized for E. coli.
SEQ ID NO: 12 is a base sequence that encodes SEQ ID NO: 6 and is codon optimized for E. coli.
SEQ ID NO: 13 represents a base sequence that encodes SEQ ID NO: 1 and is derived from Chryseobacterium nematophagum.

## Claims

1. A protein deamidase comprising a polypeptide as defined in any of (I) to (III) below:
(I) a polypeptide including (A) an amino acid sequence set forth in SEQ ID NO: 1, or (B) an amino acid sequence of positions 1 to 221 of SEQ ID NO: 1, (C) an amino acid sequence of positions 36 to 231 of SEQ ID NO: 1, or (D) an amino acid sequence of positions 36 to 221 of SEQ ID NO: 1;
(II) a polypeptide including an amino acid sequence in which one or several amino acids are substituted, added, inserted or deleted in the amino acid sequences (A) to (D), and having an activity of catalyzing a deamidation reaction of a glutamine residue of a protein; and
(III) a polypeptide including an amino acid sequence having a sequence identity of 70% or more to the amino acid sequences (A) to (D), and having an activity of catalyzing a deamidation reaction of a glutamine residue of a protein.

2. A DNA encoding the protein deamidase according to claim 1.

3. An expression cassette or a recombinant vector comprising the DNA according to claim 2.

4. A transformant obtained by transforming a host using the expression cassette or recombinant vector according to claim 3.

5. A method for producing a protein deamidase, the method comprising the step of culturing the transformant according to claim 4.

6. An enzyme preparation comprising the protein deamidase according to claim 1.

7. The enzyme preparation according to claim 6, which is a modifier for a food and drink, or a food and drink material, an industrial material, or a pharmaceutical material containing a protein.

8. A method for producing a protein in which a glutamine residue is deamidated, the method comprising the step of allowing the protein deamidase according to claim 1 to act on a protein.

9. The method according to claim 8, wherein the protein is contained in a food and drink, or a food and drink material, an industrial material, or a pharmaceutical material.

10. A method for producing a modified food and drink, or food and drink material, industrial material, or pharmaceutical material, the method comprising the step of allowing the protein deamidase according to claim 1 to act on a food and drink, or a food and drink material, an industrial material, or a pharmaceutical material containing a protein.
